# EUROPEAN PATENT SPECIFICATION

(11) **EP 4 304 694 B1**
(45) Date of publication and mention of the grant of the patent: **08.10.2025**
(21) Application number: 22768204.4
(22) Date of filing: 08.03.2022
(51) Int. Cl.: A61M 25/02, A61F 9/00, A61F 2/78, A61F 9/04, A61L 28/00, A61L 24/00, A61M 25/01

(54) **METHOD AND SYSTEM FOR MEDICAL DEVICE SECUREMENT, WOUND CLOSURE, AND WOUND/INCISION DRESSINGS**
VERFAHREN UND SYSTEM ZUR BEFESTIGUNG EINER MEDIZINISCHEN VORRICHTUNG, WUNDVERSCHLUSS UND WUND-/SCHNEIDVERBÄNDE
MÉTHODE ET SYSTÈME DE FIXATION DE DISPOSITIF MÉDICAL, FERMETURE DE PLAIE ET PANSEMENTS POUR PLAIE/INCISION

(30) Priority: 08.03.2021 US 202163157887 P
(43) Date of publication of application: 17.01.2024
(73) Proprietor: Ann and Robert H. Lurie Children's Hospital of Chicago, Chicago, IL 60611-2605 (US); Northwestern Memorial Healthcare, Chicago, IL 60611 (US)
(72) Inventor: SHARMA, Abhineet, Forest Park, Illinois 60130 (US); BRADLEY, Eric M., Huntley, Illinois 60142 (US); GRAYSON, David A., La Grange, Illinois 60525 (US); CALDARELLI, Leslie, Evanston, Illinois 60201 (US); JENSEN, James E., Wheeling, Illinois 60090 (US); TURNER, Katie L., Chicago, Illinois 60611 (US)
(74) Representative: Hancox, Jonathan Christopher
(86) International application number: PCT/US2022/071017
(87) International publication number: WO 2022/192862

(56) References cited:
- CN-A- 107 441 613
- CN-A- 107 441 613
- CN-A- 108 433 869
- CN-A- 108 433 869
- ES-U- 1 255 401
- ES-Y- 1 255 401
- JP-A- 2008 178 710
- JP-A- 2008 178 710
- US-A1- 2014 214 078

## Description

### BACKGROUND

Medical adhesives refer to various skin safe glues and other products that can be used to adhere items to the skin of a patient. As one example, a medical adhesive can be used to secure a breathing mask to a neonatal patient. Medical adhesives can also be used to attach a catheter or other ostomy appliance to a patient. In such implementations, the medical adhesive is applied to the skin of the patient, and pressure applied by the patient or physician is used to bond the mask, catheter, etc. to the patient such that a secure attachment is made. Traditional medical adhesives are available as adhesive tapes, adhesive dressings, brush-on liquids, sprays, creams, single use wipes, etc.

ES 1255401 U (Alaguero Cuervo) discloses a mask that is fixed to the face by joining neodymium magnets, characterized by being constituted by a piece of protective material to which several neodymium magnets are glued. CN 107441613 A (Wuxi Peoples Hospital) discloses a magnetic type medical catheter fixer, which comprises a bonding matrix bonded at the skin surface of a patient. A magnetic suction base is arranged on the bonding matrix, and a magnetic suction cover body is moveably arranged on the magnetic suction base. JP 2008178710 A (Nitto Denko Corp) discloses a skin sticking material comprising a flexible support film and a skin adhesive layer formed on one side of the film. CN 108433869 A (Dai Ming et al.) discloses an adhesive type magnetic eyelid closing medical device comprising an arc-shaped long-strip-shaped elastic device for attachment to the bottom of upper eyelid skin of a human eye. The device comprises a compound elastic material layer, a metal module combination layer with magnetism, an elastic bottom layer and a skin adhesion layer.

### SUMMARY

The present invention is defined by the appended claims.

An illustrative treatment system includes a first interface having a first side that includes an adhesive and a second side that includes a first magnet. The first interface is configured to attach to skin of a patient such that the first magnet does not contact the skin. The system also includes a second interface that includes a medical device and a second magnet mounted to the medical device. The second magnet is configured to magnetically bind to the first magnet such that the medical device is secured to the patient.

**In** some embodiments, the medical device is a breathing mask or other respiratory interface, and in other embodiments the medical device is an eye shield, a tube, a line, an electrode, a catheter, a cannula, a prong, a dressing that is sized to cover a wound or an incision, or a probe. In other embodiments, the second magnet is embedded within the medical device. In one embodiment, the first magnet is a first magnetic strip that conforms to a face of the patient, and the second magnet is a second magnetic strip of a same size and shape as the first magnetic strip. The adhesive can be a hydrocolloid dressing, and the first magnet can be a neodymium magnetic strip. In another embodiment, the second side of the first interface also includes the adhesive such that the first magnet is embedded within the adhesive.

An illustrative method of securing medical components includes forming a first interface that includes a first side formed from an adhesive that attaches to skin of a patient and a second side that includes a first magnet. The first interface is formed such that the first magnet does not contact the skin of the patient. The method also includes forming a second interface by mounting a second magnet to a medical device. The method further includes mounting the second interface to the first interface such that the medical device is secured to the patient.

In one embodiment, forming the second interface includes embedding the second magnet within the medical device. In another embodiment, forming the first interface includes embedding the first magnet within the adhesive. In other embodiments, the first magnet is a first magnetic strip that conforms to a face of the patient, and the second magnet is a second magnetic strip of a same size and shape as the first magnetic strip. In another embodiment, forming the second interface includes mounting the second magnet on an exterior surface of the medical device. The adhesive can be a hydrocolloid dressing, and the medical device can be a probe, an electrode, a tube, a line, a shield, or a mask.

Another illustrative treatment system includes a first interface that has a first adhesive and a first magnet attached to the first adhesive. The first interface is configured to attach to skin of a patient on a first side of a skin wound or incision. The system also includes a second interface that has a second adhesive and a second magnet attached to the second adhesive. The second interface is configured to attach to the skin of the patient on a second side of the skin wound, and the second magnet is configured to attach to the first magnet such that the skin wound is closed. In one embodiment, the first magnet is embedded within the first adhesive and the second magnet is embedded within the second adhesive. In another embodiment, the first magnet is a first magnetic strip that conforms to the first side of the skin wound, and the second magnet is a second magnetic strip of a same size and shape as the first magnetic strip.

Other principal features and advantages of the invention will become apparent to those skilled in the art upon review of the following drawings, the detailed description, and the appended claims.

### BRIEF DESCRIPTION OF THE DRAWINGS

Illustrative embodiments of the invention will hereafter be described with reference to the accompanying drawings, wherein like numerals denote like elements.
Fig. 1A is a perspective view of a magnet included in a first interface in accordance with an illustrative embodiment.
Fig. 1B is a plan view of an adhesive included in the first interface in accordance with an illustrative embodiment.
Fig. 2A depicts an external view of a mask that forms a second interface of the system in accordance with an illustrative embodiment.
Fig. 2B depicts an internal view of the mask that forms the second interface of the system in accordance with an illustrative embodiment.
Fig. 3A depicts a first interface positioned on a patient in accordance with an illustrative embodiment.
Fig. 3B is a front perspective view of the patient with the mask attached to the first interface in accordance with an illustrative embodiment.
Fig. 4A depicts an eye shield in accordance with an illustrative embodiment.
Fig. 4B depicts lateral edges of the eye shield magnetically connected to one another and central portions of the eye shield magnetically connected to one another in accordance with an illustrative embodiment.
Fig. 4C depicts the eye shield positioned behind the head of the patient and in an open configuration in accordance with an illustrative embodiment.
Fig. 4D is a view of the eye shield mounted on a patient in accordance with an illustrative embodiment.
Fig. 5A depicts a first interface on a forehead of a patient in accordance with an illustrative embodiment.
Fig. 5B is a front view of an eye patch mounted to the first interface in accordance with an illustrative embodiment.
Fig. 5C is a side view of the eye patch mounted to the first interface in accordance with an illustrative embodiment.
Fig. 6A depicts a first magnetic element mounted to a test patient for use in securement of an umbilical line in accordance with an illustrative embodiment.
Fig. 6B depicts a second magnetic element magnetically bound to the first magnetic element such that the umbilical line is secured in accordance with an illustrative embodiment.
Fig. 7A depicts a first magnetic element mounted to a test patient for use in securement of a nasogastric tube in accordance with an illustrative embodiment.
Fig. 7B depicts a second magnetic element magnetically bound to the first magnetic element such that the nasogastric tube is secured in accordance with an illustrative embodiment.

### DETAILED DESCRIPTION

The treatment and care of premature babies, which costs billions of dollars each year, often includes help with breathing and treatment for jaundice. Much of the cost is due to prolonged therapy that results from treatment complications. Traditional treatment techniques can lead to reduced effectiveness in jaundice therapy, infection, pauses in breathing, irreversible damage to skin, cartilage, and bone, death, etc. Many of these issues result from use of ventilators to assist with breathing, and to a lesser extent with eye/head coverings used to protect the neonatal during jaundice treatment. As an example, during ventilation, a neonatal mask can be used to help maintain positive pressure for the system. In one embodiment, the positive pressure can be 6-10 millimeters of water (mm H₂O), with an air flow of 10-14 liters per minute (L/min). Alternatively, other positive pressures and/or flow rates may be used. The skin to neonatal mask interface can result in injury to the skin, and the pressure used to apply the neonatal mask can result in bone and/or cartilage damage. Thus, all of the current products on the market have to balance the maintenance of a proper seal and skin breakdown. Increasing a seal puts more pressure on the neonatal skin and cartilage, causing skin breakdown and perforation. Reducing the pressure against the skin or decreasing occlusion of the nasal septum leads to a loss of proper seal, which then decreases the effectiveness of the system.

Two major issues with current treatment systems include compromised skin integrity and breakdown or the loss of sustained positive pressure due to an inadequate seal. A recent attempt to solve these problems is referred to as the Seattle PAP system. The Seattle PAP system relies on the use of a silicone interface that comes into direct contact with the neonatal skin. The system attempts to relieve some of the pressure based stress by changing the angle of the tubing apparatus that is attached to the interface. A recent randomized control trial showed that this did not improve outcomes compared to the current standard of care. In fact, both interfaces still showed greater than 30% failure rate. Other groups have tried to alternate between a mask interface and a pronged interface. While this is somewhat successful, it relies on alternating between a mask and prong. This leads to a time-frame where there is no positive pressure being delivered to a neonate, which partially defeats the purpose of continuous positive pressure systems.

A large meta analysis of 45 studies, including 14 randomized control trials, identified that groups have tried nasal barrier dressings (2 studies, n=244, risk ratio -0.12, 95%, CI - 0.20 to -0.04), nasal high flow therapy as an alternative to binasal prong continuous positive airway pressure (CPAP) (7 studies, n=1570, risk difference (RD) -0.14, 95% CI -0.17 to -0.10), and nasal masks rather than binasal prongs (5 studies, n=544, RR 0.80, 95% CI 0.64 to 1.00). However, these interventions show only marginal improvement in terms of nasal septal injury. Additionally, the use of high flow therapy is an alternative to CPAP therapy, but high flow therapy does not provide the same positive pressure that a CPAP apparatus would deliver.

Described herein is a system that includes multiple components, which are used to provide safer and more effective medical treatments. The medical treatments can include neonatal care and a wide variety of other applications, as described in more detail below. In one embodiment, the system includes a skin-safe magnet embedded adhesive, and a medical device that mounts to the adhesive. The medical device can be a magnetic strip embedded respiratory mask for positive pressure ventilation, an eye shield with magnetic elements, etc. It is believed that the proposed systems and methods overcome various barriers encountered with currently available methods and equipment, by using magnetic force to reduce the direct pressure required to secure the device for effective treatment. The proposed magnetic adhesive presents a solution to a commonly encountered problem. Currently, various equipment requires cumbersome pressure dressing that further compromises skin integrity and requires manipulation. The proposed adhesive can be used to secure various other devices, such as eye covers for neonatal phototherapy, osteotomy bags, and surgical silos. Of these, the eye shield represents a unique opportunity as almost 50% of neonates require phototherapy, and the traditionally used shields reduce the effectiveness of phototherapy.

In one embodiment, the proposed system uses one or more neodymium magnetic elements mounted on or embedded into materials to create a safe latching interface between two surfaces. Alternatively, other types of magnets can be used such as a Samarium-cobalt rare earth magnet, a permanent magnet, etc. The first component of the system is a magnetic adhesive, which in one embodiment can include a hydrocolloid dressing applied to a patient's skin. Any type of hydrocolloid material or dressing can be used. Such hydrocolloid dressings are known to be safe for neonatal skin and can be used for a variety of applications in neonatal medicine. The hydrocolloid dressing can be used to prevent skin breakdown, and also acts as an adhesive. The opposite side of the hydrocolloid dressing of the interface contains a thin neodymium magnet. In alternative embodiments, a plurality of magnets may be used instead of a single strip of magnetic material. The magnetic element can either be on top of the hydrocolloid component or sandwiched between two hydrocolloid layers. Therefore, in an illustrative embodiment, the magnetic element is positioned such that it does not come into contact with the skin. In an alternative embodiment, the magnetic element can be fully integrated/embedded into the hydrocolloid during the manufacturing process.

Also described herein are masks, nasal prongs, and cannula embedded with a thin magnetic strip that binds to the magnet-embedded skin adhesive interface. Although several embodiments herein reference neonatal devices, it is important to understand that the proposed masks, nasal prongs, and cannula can be of any size to fit any human or animal, regardless of their age or size. In an illustrative embodiment, the mask component includes a silicone mask with a neodymium magnetic strip embedded against the internal surface. In alternative embodiments, different materials may be used to form the mask. The mechanism allows two magnetic components to latch together and conform to a neonate's nasal bone while allowing for positive-pressure delivery. Currently, there are significant issues with commercially available products. From the standpoint of the mask, currently available systems are not secure and lead to pressure leaks around the interface, causing variable delivery of positive pressure to the lungs. Conversely, the proposed magnetic mechanism forms a tight seal that does not rely on downward pressure and multiple straps to maintain its seal and position. The proposed mask provides an interface to deliver respiratory support without causing pressure breakdown of the skin or nasal bone.

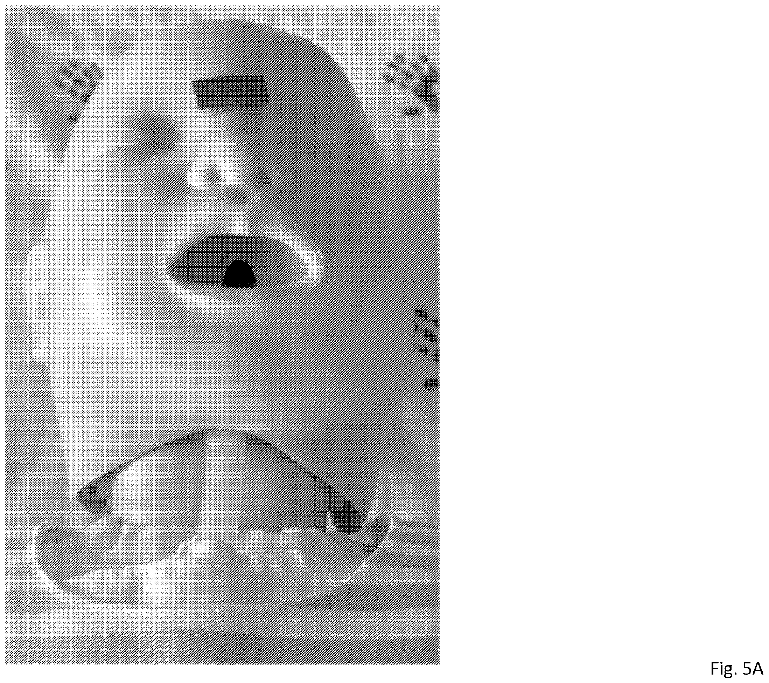

The magnetic interface can similarly be used to secure an eye shield to the neonate during light treatment for j aundice. The eye shield can include magnetic elements against its lateral edge and a medial strip that allows the shield to be more easily secured while reducing the size profile of the mask. The magnetic features allow two surfaces to latch using magnetic polarity instead of tight pressure effectively. The magnetic features also cover less surface area (e.g., Figs. 5A-5C), making phototherapy more effective and decrease the risk of brain bleed from manipulating a strap around the head. This same adhesive technology can also be used to secure other medical devices, and can also be used to perform negative pressure wound therapy dressing adhesion. The magnetic adhesive can also be used for other wound dressings to form a boundary along a wound such that a dressing in the form of a magnetic cover can be applied to protect the wounded area. This approach would offer protection to the patient, while allowing the physician to repeatedly access and assess the wound with reduced discomfort for the patient.

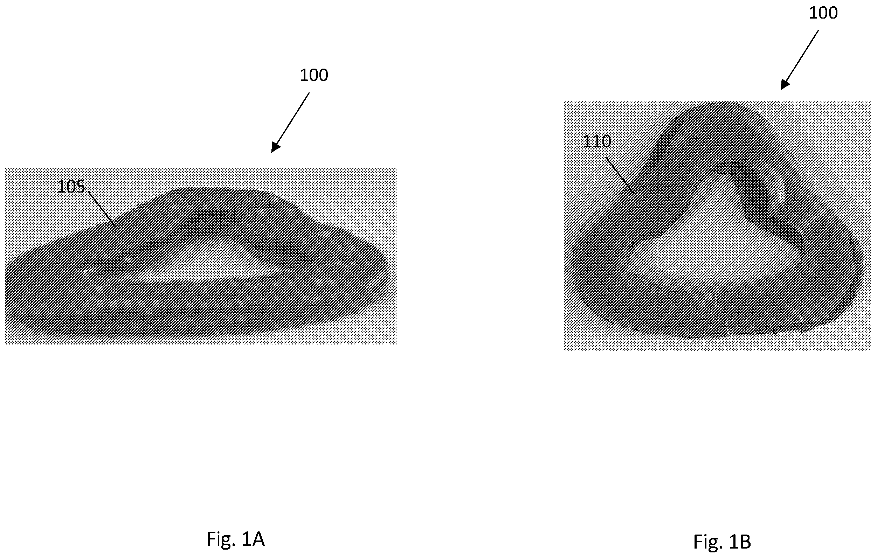

A more detailed discussion of the system components is included below. Figs. 1A and 1B depicts a first interface that includes a magnetic strip attached to an adhesive. Specifically, Fig. 1A is a perspective view of a magnet 105 included in the first interface 100 in accordance with an illustrative embodiment. Fig. 1B is a plan view of an adhesive 110 included in the first interface 100 in accordance with an illustrative embodiment. As shown, the first interface 100 is shaped to accommodate a nose of a patient. In alternative embodiments, the first interface 100 can have a different shape and/or size. Also, as discussed herein, the first interface can be sized/shaped for numerous other purposes such as securing tubes, lines, masks, shields, bags, electrodes, and sensors, sealing wounds, etc. As shown, a first side of the first interface 100 includes the adhesive 110 and a second side (opposite the first side) of the interface includes the magnet 105.

The adhesive used to form the first interface can be a hydrocolloid dressing (e.g., Duoderm, Tegaderm, Comfeel Plus, Medihoney, Restore, Replicare, Nuderm, etc.), an absorptive/foam dressing (e.g., Mepilex), or a transparent film (e.g., Tegaderm) that contacts and adheres to the skin of the patient. As shown in Figs. 1A and 1B, the magnetic strip can be on an opposite side of the adhesive such that the magnetic strip does not contact the skin of the patient. Additionally, in some embodiments, the magnetic strip can be surrounded by the hydrocolloid dressing. For example, in one embodiment, the magnetic strip can be placed in between first and second layers of the hydrocolloid dressing, or embedded completely during the manufacturing process.

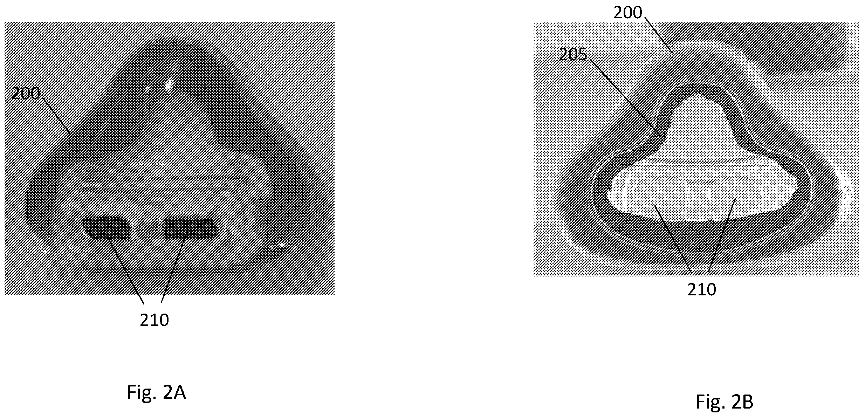

Fig. 2A depicts an external view of a mask 200 that forms a second interface of the system in accordance with an illustrative embodiment. Fig. 2B depicts an internal view of the mask 200 that forms the second interface of the system in accordance with an illustrative embodiment. The mask includes a magnetic strip 205 that is sized and shaped to match or substantially match the magnetic strip of the first interface. In an illustrative embodiment, the magnetic strip can be integrally mounted within the material that forms the mask, as shown in Fig. 2B. The magnetic components can also be embedded completely into the mask during the manufacturing process. Alternatively, the magnetic strip 205 can be mounted to the mask using an adhesive in one embodiment. In such an embodiment, the magnetic strip 205 may be positioned on an external surface of the mask 200. In one embodiment, the mask can be formed of silicon and the magnetic strip can be a neodymium strip. Alternatively, a different biocompatible material may be used for the mask and/or a different type of magnet may be used. The magnetic strip in the mask is oriented such that it is magnetically attracted to the magnet of the first interface that is placed onto the patient (i.e., such that a polarity of an outward facing portion of the magnetic strip 205 is opposite of the polarity of an outward facing portion of the magnet of the first interface). As shown in both Figs. 2A and 2B, the mask includes a pair of openings 210 that are sized to receive breathing tubes.

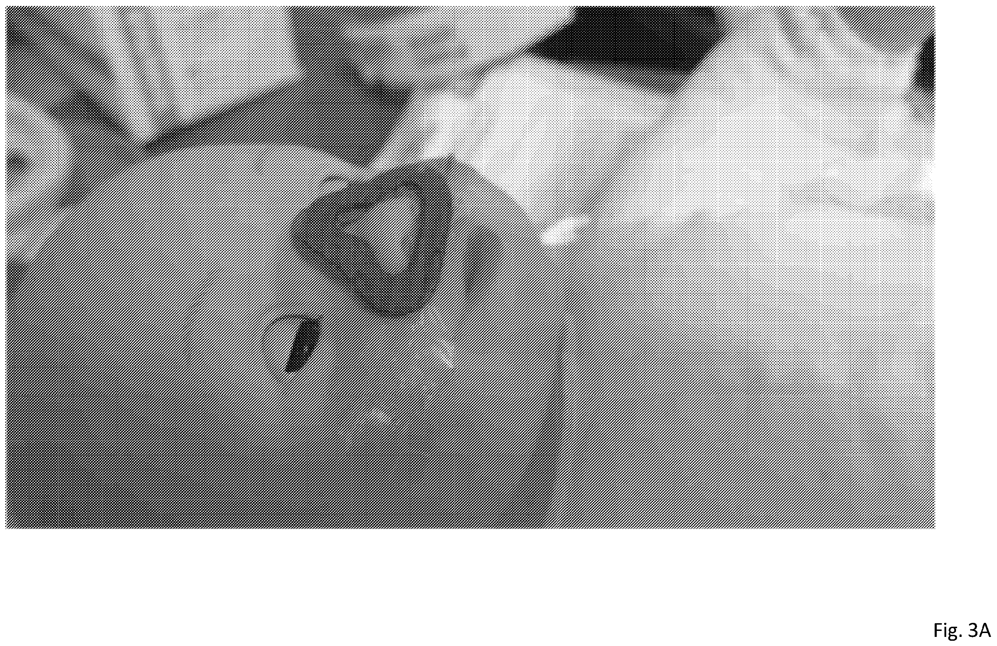

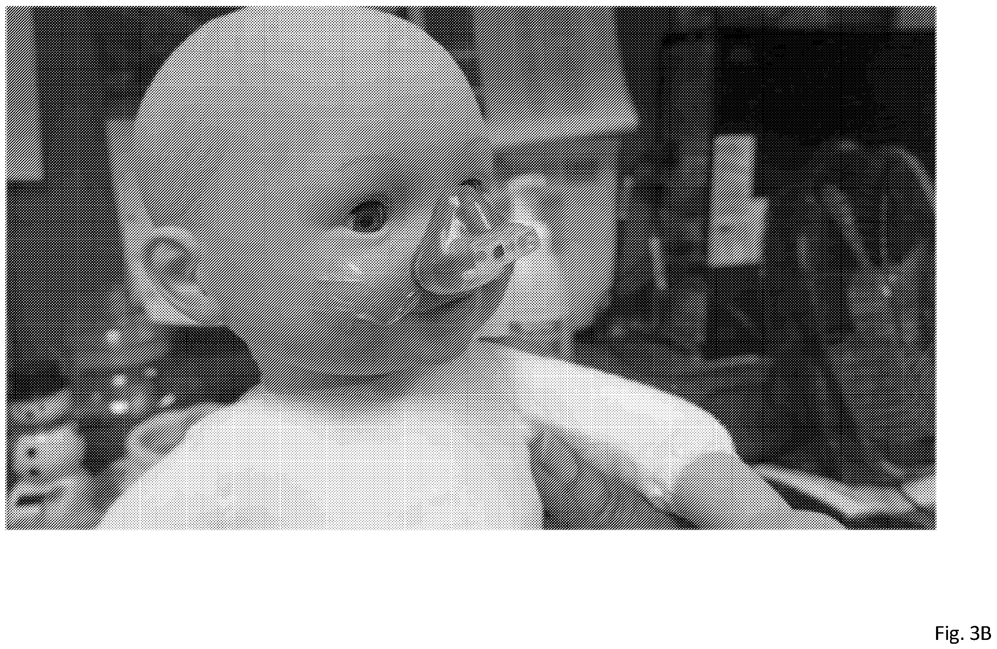

Fig. 3A depicts a first interface positioned on a patient in accordance with an illustrative embodiment. As shown, the dressing conforms around the nose of the patient. Fig. 3B is a front perspective view of the patient with the mask attached to the first interface in accordance with an illustrative embodiment. As shown, both the magnetic strip in the first interface and the mated magnetic strip in the mask do not make any contact with the skin of the patient. Additionally, unlike traditional systems, the mask can effectively latch on to the first interface using the opposite magnetic fields instead of downward pressure from a strap.

As discussed, preterm neonates undergoing phototherapy for jaundice require eye protection. Currently, eye protection is secured using cumbersome straps that require frequent manipulation. The techniques described herein can be used to secure an eye shield to the patient with magnetic elements that can be secured to one another and/or to a small strip of magnetic adhesive on the patient.

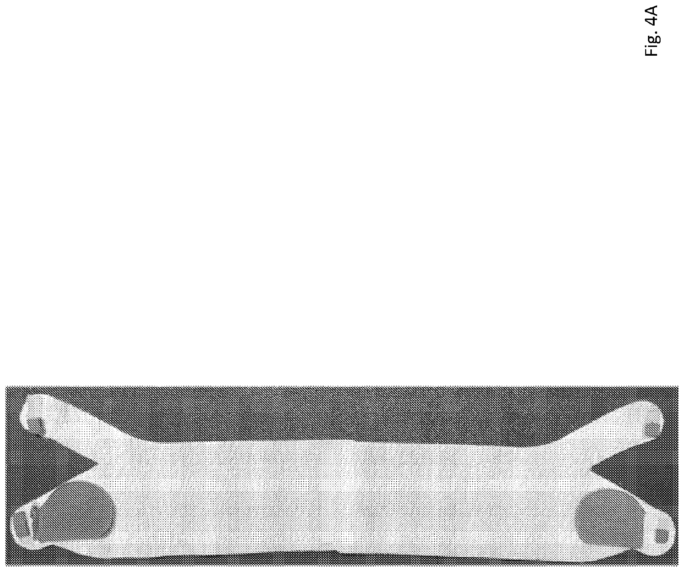

Fig. 4A depicts an eye shield in accordance with an illustrative embodiment. As shown, the eye shield includes a strap that wraps around the head of the patient, and a pair of eye patches are attached to an internal side of the strap. The strap also includes lateral edges and a central portion, each of which include magnets mounted thereon. In some embodiments, a first interface is placed on the patient and the magnets on the lateral edges of the eye shield attach to the first interface. Alternatively, the lateral edges of the eye shield can attach to one another. A central portion of the eye shield also includes two magnets that attach to one another, and allow for easy detachment from and reattachment to the patient. The eye patches are designed to be positioned over the patient's eyes when the eye shield is placed around the head of the patient.

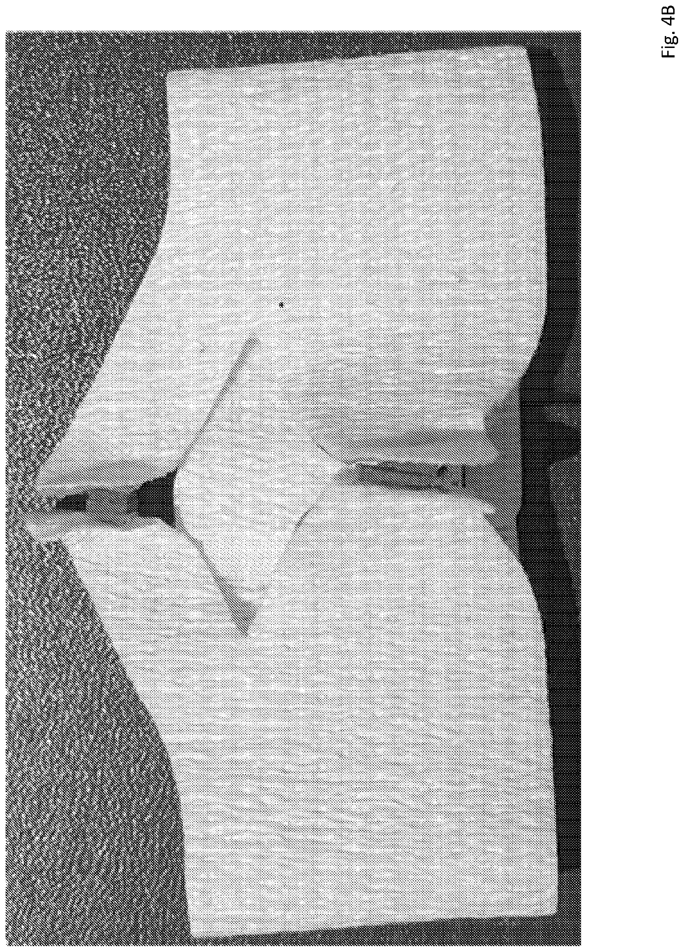

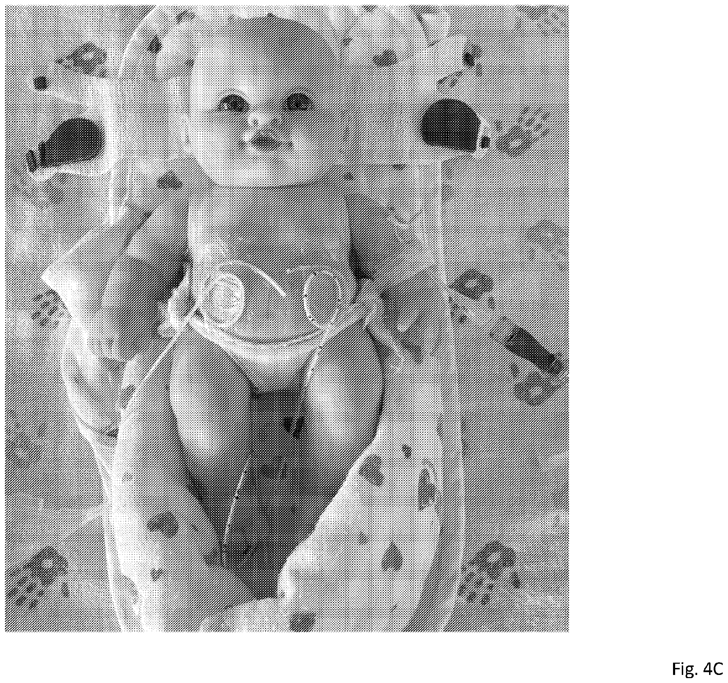

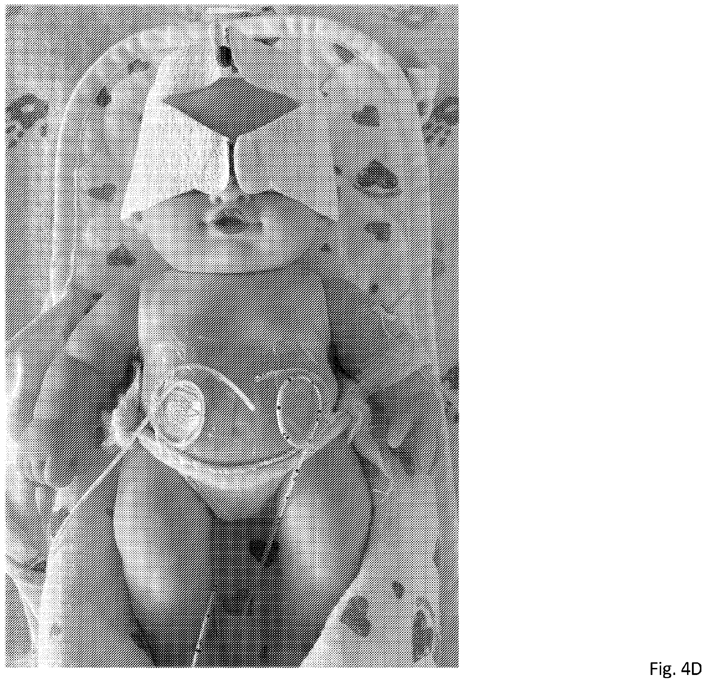

Fig. 4B depicts lateral edges of the eye shield magnetically connected to one another and central portions of the eye shield magnetically connected to one another in accordance with an illustrative embodiment. As shown, the strap that forms the eye shield has a diamond shaped opening when the lateral edges and central portions are connected. Fig. 4C depicts the eye shield positioned behind the head of the patient and in an open configuration in accordance with an illustrative embodiment. Fig. 4D is a view of the eye shield mounted on a patient in accordance with an illustrative embodiment.

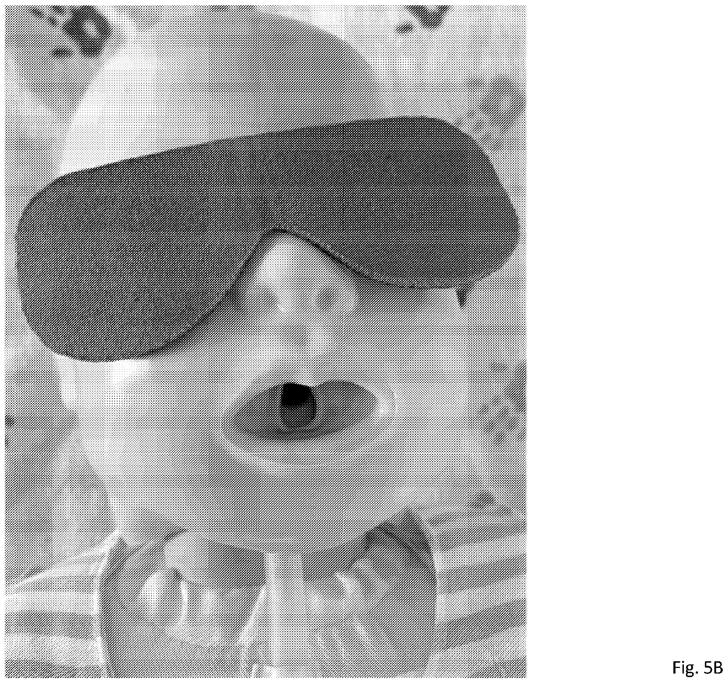

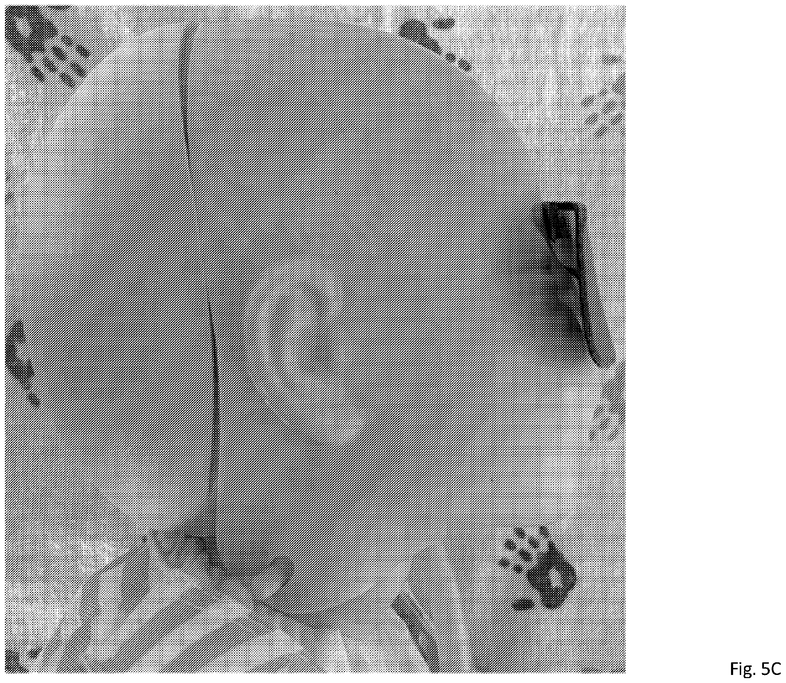

In another embodiment, instead of using a strap that wraps around the head, the eye shield can have an eyeglasses shape and mount directly to the patient's head using the techniques described herein. Fig. 5A depicts a first interface on a forehead of a patient in accordance with an illustrative embodiment. The first interface includes an adhesive adjacent to the patient's forehead and a magnet secured to the adhesive such that the magnet does not contact the skin of the patient. The eye patch includes a magnet that attaches to the magnet of the first interface. Fig. 5B is a front view of an eye patch mounted to the first interface in accordance with an illustrative embodiment. Fig. 5C is a side view of the eye patch mounted to the first interface in accordance with an illustrative embodiment. As shown, the eye patch is shaped to fit over the patient's eyes to block any light used during treatment. This embodiment reduces the total surface area that is covered by the eye shield, thereby increasing phototherapy effectiveness. It also reduces the need to manipulate the neonate's head and risk a brain bleed when applying and removing the eye shield.

The methods and systems described herein provide significant economic value to healthcare institutions. For example, there is cost savings for an intensive care unit by maintaining a patient on noninvasive ventilation using the proposed system, as opposed to invasive ventilation via a ventilator. Additionally, the materials cost for the proposed system represents a savings over the cost of the current apparatuses that are used. The proposed system also uses less plastic and textiles than traditional systems. The proposed design would also require less frequent replacement due to manipulation. Additionally, CPAP tolerance is well established to lower hospital days for neonates. One of the critical causes for CPAP failure is the inability to tolerate the current mask due to nasal septal breakdown or inadequate delivery of positive pressure. A large meta-analysis recently showed that nasal injury in preterm infants ranged from 20-100%. Additionally, infants born at less than 30 weeks of gestation are at the highest risk. Furthermore, nasal cartilage breakdown is permanent and can require surgical intervention if severe. The proposed system and techniques help to alleviate these problems.

In addition to the neonatal applications described herein, there are also various other applications for the proposed magnetic adhesive. The magnetic adhesive can be used for other purposes, such as securing medical devices and equipment. As discussed in more detail below, the proposed methods and techniques described herein can further be used in CPAP masks, non-invasive ventilation, respiratory support, eye shields, skin safe magnetic adhesive, etc.

In one embodiment, the proposed methods and systems can be used for neonatal, pediatric, and/or adult respiratory care including mask securing in CPAP applications, mask securing in bi-level positive airway pressure (BiPAP) applications, securing of a low flow or high flow nasal cannula, a tracheostomy tube securement, an endotracheal tube (ETT) securement, etc. Depending on the type of respiratory treatment, the proposed methods and systems may be used at home or as inpatient treatments at a medical facility. As compared to traditional systems, the proposed system is safer and more efficient because it is easier to secure quickly, detach, and reposition, with reduced risk of injury from prolonged pressure application on the back of the neck (e.g., tracheostomy tube), and reduced risk of injury on the face (e.g., endotracheal tube).

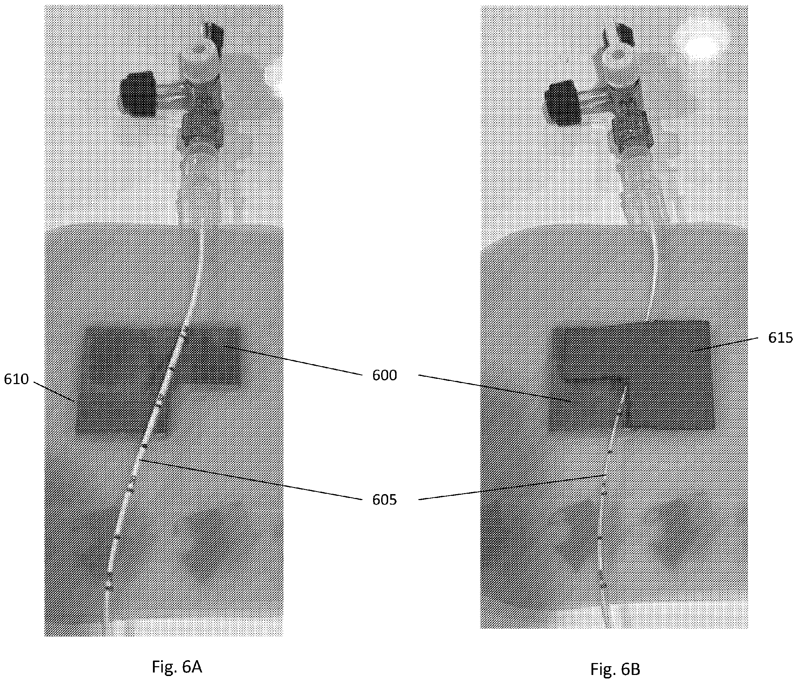

Fig. 6A depicts a first magnetic element 600 mounted to a test patient for use in securement of an umbilical line 605 in accordance with an illustrative embodiment. As shown, the first magnetic element 600 is mounted via an adhesive 610, as described herein. Fig. 6B depicts a second magnetic element 615 magnetically bound to the first magnetic element 600 such that umbilical line 605 is secured in accordance with an illustrative embodiment.

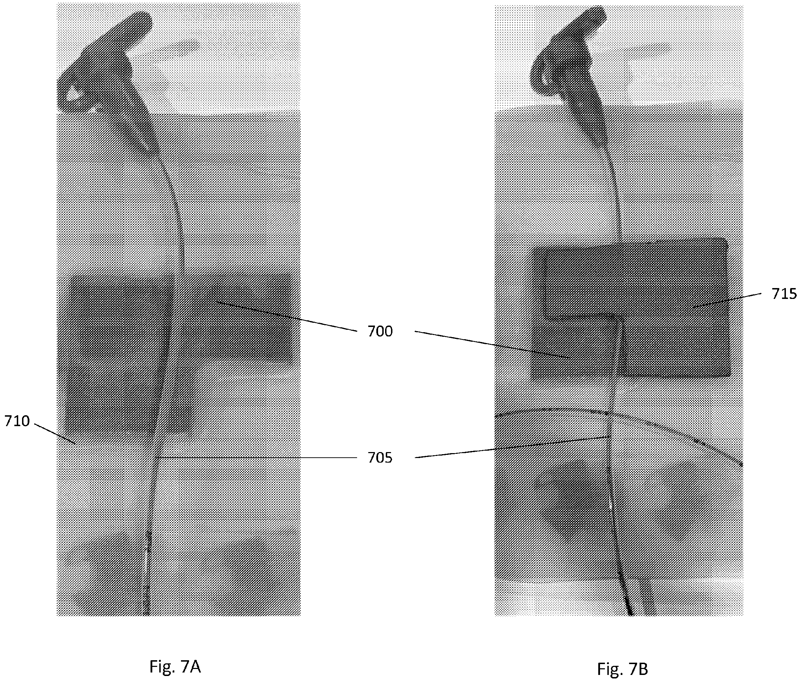

Fig. 7A depicts a first magnetic element 700 mounted to a test patient for use in securement of a nasogastric tube 705 in accordance with an illustrative embodiment. As shown, the first magnetic element 700 is mounted via an adhesive 710, as described herein. Fig. 7B depicts a second magnetic element 715 magnetically bound to the first magnetic element 700 such that the nasogastric tube 705 is secured in accordance with an illustrative embodiment.

The proposed methods and systems can also be used for line and tube securement applications such as peripheral intravenous (PIV) line securement, central line securement, umbilical line securement, A-line securement, nasogastric/nasojejunal tube securement, chest tube securement, etc. These types of line securements are typically formed as inpatient treatments at a medical facility. As compared to traditional systems, the proposed system is safer and more efficient in securing lines and tubes because it is easier to secure quickly and adjust, and because it reduces the risk of injury (e.g., reduced risk of pressure injury on nares in nasogastric/nasojejunal tube securement).

The proposed methods and systems can also be used to help secure monitoring equipment to a patient, such as SpO₂ monitoring equipment (i.e., equipment that monitors the amount of oxygen in a patient's blood), near-infrared spectroscopy (NIRS) monitoring equipment, electroencephalogram (EEG) monitoring equipment, electrocardiogram (EKG) (e.g., 12-lead) monitoring equipment, telemetry (e.g., 3/5 lead) monitoring equipment, etc. These types of monitoring equipment are generally used in an inpatient, critical care, or neurological treatment setting. As compared to traditional systems, the proposed system is safer and more efficient in securing monitoring equipment because it is easier to secure and detach quickly.

The proposed methods and systems can also be used to perform incision/wound care for patients, such as magnetic sutures to perform a suture free wound closure. For example, a flesh wound (e.g., cut, laceration, gouge, etc.) in the skin can be sutured using the proposed system. A first interface is formed and attached to the skin on a first side of the skin wound. The first interface includes a first adhesive and a first magnet attached to the first adhesive. Similarly, a second interface is formed and attached to the skin on a second side of the skin wound (i.e., opposite the first side). The second interface includes a second adhesive and a second magnet attached to the second adhesive. The polarities of the first and second magnets are arranged such that the second magnet is configured to attach to the first magnet to close the skin wound and allow healing to occur.

As compared to traditional systems, the use of magnetic sutures allows for easier securing of the wound (especially on mobile pediatric patients) and for easier adjustments of the closure as the wound heals/evolves. The proposed methods and systems can also be used to perform wound vac (i.e., vacuum assisted closure of a wound), wound/drain dressings, wound/prevention dressings, surgical seals, surgical silos, etc. As compared to traditional systems, the use of the proposed system for these procedures results in easier attachment/detachment for wound and site assessment. For example, as discussed with respect to wound/incision dressings, a first magnet/dressing is on the skin creating a border around the wound/incision, and the other magnet interfaces with the first magnet and attaches to the dressing that covers the entire wound/incision. Also, as used herein, a surgical seal can be anything to augment closure of a site either as the primary method or an adjunct. For example, it could be something to augment skin approximation and closure after laceration. These incision/wound applications can be performed as inpatient or at home procedures.

As discussed, the proposed methods and systems can be used to secure a phototherapy eye shield to a neonatal patient, which provides easier attachment/detachment and more secure positioning as compared to traditional techniques. The proposed methods and systems can also be used in temperature monitoring of neonatal, pediatric, and adult patients and enables closed loop temperature control in incubators and radiant warmers, while providing easy attachment/detachment of the temperature probes. The system can also be used for maternal health such as tocodynamometry (i.e., obstetric care) and external fetal heart monitoring, in which the system is easier to attach/detach and provides more precise positioning than traditional systems, provides more security that the currently used strap, and enables mobility in labor.

The proposed system can further be used in various miscellaneous procedures such as indwelling urinary catheter securement to the patient's leg (inpatient/outpatient), in which the system is much easier to adjust and reduces the risk of pressure injury as compared to traditional systems. The system can be used in active cooling systems (critical care) to provide easy attachment/detachment of cooling pads (e.g., Arctic Sun). The system can further be used in ostomy bags to provide easy attachment/detachment for cleaning, assessment, bag changes, etc.

The word "illustrative" is used herein to mean serving as an example, instance, or illustration. Any aspect or design described herein as "illustrative" is not necessarily to be construed as preferred or advantageous over other aspects or designs. Further, for the purposes of this disclosure and unless otherwise specified, "a" or "an" means "one or more."

The foregoing description of illustrative embodiments of the technology has been presented for purposes of illustration and of description. It is not intended to be exhaustive or to limit the technology to the precise form disclosed, and modifications and variations are possible in light of the above teachings or may be acquired from practice of the technology. The embodiments were chosen and described in order to explain the principles of the technology and as practical applications of the invention to enable one skilled in the art to utilize the technology in various embodiments and with various modifications as suited to the particular use contemplated. It is intended that the scope of the invention be defined by the claims appended hereto.

## Claims

1. A treatment system comprising:
a first interface (100) having a first side that includes an adhesive (110, 610, 710) and a second side that includes a first magnet (105, 600, 700), wherein the first interface is configured to attach to skin of a patient such that the first magnet does not contact the skin, and wherein the second side of the first interface also includes the adhesive such that the first magnet is embedded within the adhesive; and
a second interface that includes a medical device (200, 605, 705) and a second magnet (205, 615, 715) mounted to the medical device, wherein the second magnet is configured to magnetically bind to the first magnet such that the medical device is secured to the patient.

2. The treatment system of claim 1, wherein the medical device (200, 605, 705) comprises a breathing mask.

3. The treatment system of claim 1, wherein the second magnet (205, 615, 715) is embedded within the medical device (200, 605, 705).

4. The treatment system of claim 1, wherein the first magnet (105, 600, 700) comprises a first magnetic strip that conforms to a face of the patient, and wherein the second magnet (205, 615, 715) comprises a second magnetic strip of a same size and shape as the first magnetic strip.

5. The treatment system of claim 1, wherein the adhesive (110, 610, 710) comprises a hydrocolloid dressing.

6. The treatment system of claim 1, wherein the first magnet (105, 600, 700) comprises a neodymium magnetic strip.

7. The treatment system of claim 1, wherein the medical device (200, 605, 705) comprises an eye shield or a dressing that is sized to cover a wound or an incision.

8. The treatment system of claim 1, wherein the medical device (200, 605, 705) comprises a tube, a line, or a catheter.

9. The treatment system of claim 1, wherein the medical device (200, 605, 705) comprises an electrode or a probe.

10. A method of securing medical components, the method comprising:
forming a first interface (100), wherein the first interface includes a first side formed from an adhesive (110, 610, 710) that attaches to skin of a patient and a second side that includes a first magnet (105, 600, 700), wherein the first interface is formed such that the first magnet does not contact the skin of the patient, and wherein forming the first interface comprises embedding the first magnet within the adhesive;
forming a second interface by mounting a second magnet (205, 615, 715) to a medical device (200, 605, 705); and
mounting the second interface to the first interface such that the medical device is secured to the patient.

11. The method of claim 10, wherein forming the second interface comprises embedding the second magnet (205, 615, 715) within the medical device (200, 605, 705).

12. The method of claim 10, wherein the first magnet (105, 600, 700) comprises a first magnetic strip that conforms to a face of the patient, and wherein the second magnet (205, 615, 715) comprises a second magnetic strip of a same size and shape as the first magnetic strip.

13. The method of claim 10, wherein forming the second interface comprises mounting the second magnet (205, 615, 715) on an exterior surface of the medical device (200, 605, 705).

## Patentansprüche

1. Behandlungssystem, umfassend:
eine erste Schnittstelle (100) mit einer ersten Seite, die einen Klebstoff (110, 610, 710) enthält, und einer zweiten Seite, die einen ersten Magneten (105, 600, 700) enthält, wobei die erste Schnittstelle dazu konfiguriert ist, an der Haut eines Patienten zu haften, sodass der erste Magnet die Haut nicht berührt, und wobei die zweite Seite der ersten Schnittstelle auch den Klebstoff enthält, sodass der erste Magnet in den Klebstoff eingebettet ist; und
eine zweite Schnittstelle, die eine medizinische Vorrichtung (200, 605, 705) und einen zweiten Magneten (205, 615, 715) enthält, der an der medizinischen Vorrichtung montiert ist, wobei der zweite Magnet dazu konfiguriert ist, sich magnetisch an den ersten Magneten zu binden, sodass die medizinische Vorrichtung an dem Patienten befestigt ist.

2. Behandlungssystem nach Anspruch 1, wobei die medizinische Vorrichtung (200, 605, 705) eine Atemmaske umfasst.

3. Behandlungssystem nach Anspruch 1, wobei der zweite Magnet (205, 615, 715) in die medizinische Vorrichtung (200, 605, 705) eingebettet ist.

4. Behandlungssystem nach Anspruch 1, wobei der erste Magnet (105, 600, 700) einen ersten Magnetstreifen umfasst, der sich an ein Gesicht des Patienten anpasst, und wobei der zweite Magnet (205, 615, 715) einen zweiten Magnetstreifen einer gleichen Größe und Form wie der erste Magnetstreifen umfasst.

5. Behandlungssystem nach Anspruch 1, wobei der Klebstoff (110, 610, 710) einen Hydrokolloidverband umfasst.

6. Behandlungssystem nach Anspruch 1, wobei der erste Magnet (105, 600, 700) einen Neodym-Magnetstreifen umfasst.

7. Behandlungssystem nach Anspruch 1, wobei die medizinische Vorrichtung (200, 605, 705) eine Augenbinde oder einen Verband umfasst, der bemessen ist, um eine Wunde oder einen Einschnitt abzudecken.

8. Behandlungssystem nach Anspruch 1, wobei die medizinische Vorrichtung (200, 605, 705) einen Schlauch, eine Leitung oder einen Katheter umfasst.

9. Behandlungssystem nach Anspruch 1, wobei die medizinische Vorrichtung (200, 605, 705) eine Elektrode oder eine Sonde umfasst.

10. Verfahren zum Befestigen medizinischer Komponenten, wobei das Verfahren Folgendes umfasst:
Ausbilden einer ersten Schnittstelle (100), wobei die erste Schnittstelle eine erste Seite, die aus einem Klebstoff (110, 610, 710) ausgebildet ist, der an der Haut eines Patienten haftet, und eine zweite Seite, die einen ersten Magneten (105, 600, 700) enthält, enthält, wobei die erste Schnittstelle so ausgebildet ist, dass der erste Magnet die Haut des Patienten nicht berührt, und wobei das Ausbilden der ersten Schnittstelle Einbetten des ersten Magneten in den Klebstoff umfasst;
Ausbilden einer zweiten Schnittstelle durch Montieren eines zweiten Magneten (205, 615, 715) an einer medizinischen Vorrichtung (200, 605, 705); und
Montieren der zweiten Schnittstelle an der ersten Schnittstelle, sodass die medizinische Vorrichtung an dem Patienten befestigt ist.

11. Verfahren nach Anspruch 10, wobei das Ausbilden der zweiten Schnittstelle Einbetten des zweiten Magneten (205, 615, 715) in die medizinische Vorrichtung (200, 605, 705) umfasst.

12. Verfahren nach Anspruch 10, wobei der erste Magnet (105, 600, 700) einen ersten Magnetstreifen umfasst, der sich an ein Gesicht des Patienten anpasst, und wobei der zweite Magnet (205, 615, 715) einen zweiten Magnetstreifen einer gleichen Größe und Form wie der erste Magnetstreifen umfasst.

13. Verfahren nach Anspruch 10, wobei das Ausbilden der zweiten Schnittstelle Montieren des zweiten Magneten (205, 615, 715) an einer äußeren Oberfläche der medizinischen Vorrichtung (200, 605, 705) umfasst.

## Revendications

1. Système de traitement comprenant :
une première interface (100) comportant un premier côté qui comprend un adhésif (110, 610, 710) et un second côté qui comprend un premier aimant (105, 600, 700), ladite première interface étant conçue pour se fixer à la peau d'un patient de sorte que le premier aimant n'entre pas en contact avec la peau, et ledit second côté de la première interface comprenant également l'adhésif de sorte que le premier aimant soit incorporé à l'intérieur de l'adhésif ; et
une seconde interface qui comprend un dispositif médical (200, 605, 705) et un second aimant (205, 615, 715) monté sur le dispositif médical, ledit second aimant étant conçu pour se lier magnétiquement au premier aimant de sorte que le dispositif médical soit fixé au patient.

2. Système de traitement selon la revendication 1, ledit dispositif médical (200, 605, 705) comprenant un masque respiratoire.

3. Système de traitement selon la revendication 1, ledit second aimant (205, 615, 715) étant incorporé à l'intérieur du dispositif médical (200, 605, 705).

4. Système de traitement selon la revendication 1, ledit premier aimant (105, 600, 700) comprenant une première bande magnétique qui se conforme au visage du patient, et ledit second aimant (205, 615, 715) comprenant une seconde bande magnétique de même taille et de même forme que la première bande magnétique.

5. Système de traitement selon la revendication 1, ledit adhésif (110, 610, 710) comprenant un pansement hydrocolloïde.

6. Système de traitement selon la revendication 1, ledit premier aimant (105, 600, 700) comprenant une bande magnétique de néodyme.

7. Système de traitement selon la revendication 1, ledit dispositif médical (200, 605, 705) comprenant une protection oculaire ou un pansement qui est dimensionné de manière à recouvrir une plaie ou une incision.

8. Système de traitement selon la revendication 1, ledit dispositif médical (200, 605, 705) comprenant un tube, une ligne ou un cathéter.

9. Système de traitement selon la revendication 1, ledit dispositif médical (200, 605, 705) comprenant une électrode ou une sonde.

10. Procédé de fixation de composants médicaux, le procédé comprenant :
la formation d'une première interface (100), ladite première interface comprenant un premier côté formé à partir d'un adhésif (110, 610, 710) qui se fixe à la peau d'un patient et un second côté qui comprend un premier aimant (105, 600, 700), ladite première interface étant formée de sorte que le premier aimant n'entre pas en contact avec la peau du patient, et la formation de la première interface comprenant l'incorporation du premier aimant à l'intérieur de l'adhésif ;
la formation d'une seconde interface en montant un second aimant (205, 615, 715) sur un dispositif médical (200, 605, 705) ; et
le montage de la seconde interface sur la première interface de sorte que le dispositif médical soit fixé au patient.

11. Procédé selon la revendication 10, ladite formation de la seconde interface comprenant l'incorporation du second aimant (205, 615, 715) à l'intérieur du dispositif médical (200, 605, 705).

12. Procédé selon la revendication 10, ledit premier aimant (105, 600, 700) comprenant une première bande magnétique qui se conforme au visage du patient, et ledit second aimant (205, 615, 715) comprenant une seconde bande magnétique de même taille et de même forme que la première bande magnétique.

13. Procédé selon la revendication 10, ladite formation de la seconde interface comprenant le montage du second aimant (205, 615, 715) sur une surface externe du dispositif médical (200, 605, 705).
